# EUROPEAN PATENT SPECIFICATION

(11) **EP 4 239 106 B1**
(45) Date of publication and mention of the grant of the patent: **04.06.2025**
(21) Application number: 22382193.5
(22) Date of filing: 02.03.2022
(51) Int. Cl.: C25B 15/08, C25B 15/021, C25B 13/08, C25B 13/07, C25B 9/70, C25B 9/23, C25B 1/23, C25B 1/042, C25B 1/04, C01B 13/10, B01D 53/62, C07C 29/151, C01B 3/02

(54) **E-METHANOL INSTALLATION PLANT**
E-METHANOL-INSTALLATIONSANLAGE
USINE D'E-MÉTHANOL

(43) Date of publication of application: 06.09.2023
(73) Proprietor: Iberdrola Clientes, S.A.U, 48009 Bilbao (Bizkaia) (ES); FORESA, INDUSTRIAS QUÍMICAS DEL NOROESTE, S.A.U., 36650 Caldas de Reis (Pontevedra) (ES)
(72) Inventor: Fúnez Guerra, Carlos, 48009 Bilbao (BIZKAIA) (ES); Lobato Elosegui, Iñigo, 48009 Bilbao (ES); Riveiro Currais, Manolo, Negreira (ES); Brandon Pereira, Jose Manuel, Caldas de Reis (ES)
(74) Representative: Pons

(56) References cited:
- EP-A1- 3 957 773
- US-A1- 2009 289 227

## Description

### OBJECT OF THE INVENTION

The present invention relates to the field of e-methanol installation plants of the type that are directly fed by renewable power source without electrical power storage means. More particularly, it relates to a methanol installation plant comprising a carbon capture module from biomass boiler and from the air, a plurality of electrolysers, heat recovering system, water circularity and a methanol synthesis reactor directly fed with syngas and a mixture of hydrogen and captured carbon dioxide to produce methanol according to a Ficher-Tropsh process.

### BACKGROUND OF THE INVENTION

Methanol production installations integrated with renewable energies are widely known in the state of the art.

Notwithstanding, known methanol installation plants are often connected to the grid in order to achieve a steady methanol production. In addition to that, it is known in the state of the art to make use of a sole electrolyser, either a PEM, an alkaline, SOEC and CO-SOEC. The use of only one type of electrolyser, or even two, derives in less flexibility, less services and less efficiency.

In some known methanol installations, the production of syngas is achieved by a previous step of producing hydrogen from the natural gas produced in steam methane reforming and with carbon dioxide captured. This allows to later separate CO2 and hydrogen from the syngas produced, and then to introduce them as inputs in a methanol reactor. This configuration incurs in an important amount of carbon dioxide emissions.

For example, document GB2552010 discloses a method for synthesizing carbon compounds comprising the steps of generating electricity from a renewable source; recovering carbon dioxide from the atmosphere; providing a supply of hydrogen; and reacting the recovered carbon dioxide with the hydrogen in an exothermic reaction; in which the step of recovering the carbon dioxide is powered by electricity and by thermal energy. This document intends to solve the availability of renewable energies by thermic storage and exploring possibilities of phase-change materials.

In contrast, document EP2049232A1 address the problem of renewable intermittency and integration with electrolysers by proposing a SOEC electrolyser capable of maintaining the temperature without having to reverse it into an SOFC fuel cell and/or without having to burn H2 or syngas fuel.

Document WO2021102503A1 relates to a renewable methanol production module comprising a water capture generator designed for directly capturing water from the atmosphere to provide water in a liquid form, an electrolyser coupled to the water capture generator for receiving the liquid water, the electrolyser being effective in electrolysis of the liquid to produce hydrogen, a reactor coupled to the electrolyser for receiving the hydrogen and reacting it with carbon dioxide to produce renewable methanol. It is therein described that carbon dioxide may be recovered from flue gas using Carbon Capture and Storage (CCS) technology.

### DESCRIPTION OF THE INVENTION

According to a first aspect of the invention it is described a e-methanol production installation, a carbon capture module operatively connected with the biomass boiler for capturing CO2 from said stream of exhaust gases, a carbon capturing device, optionally a Direct Air Capture system (DAC) and/or enzyme based, a hybridization of electrolysers technology systems electrically connected with a source of renewable energy and an advance methanol synthesis process based in Fischer Tropsch process occurring in a methanol synthesis reactor.

More particularly, the electrolysers module in turn comprises:
- a solid oxide co-electrolyser fed with the captured CO2, renewable electricity, and steam, producing an outlet flow of syngas, optionally at 1.5 barg,
- a solid oxide electrolyser (SOEC) fed with steam and renewable electricity, producing an outlet flow of hydrogen, optionally at 1.5 barg,
- a proton exchange membrane (PEM) electrolyser fed with water and renewable electricity producing a second outlet flow of hydrogen, optionally at 30 barg,
- an alkaline electrolyser fed with water and renewable electricity producing a third outlet flow of hydrogen at, optionally, 5 barg.

The invention further provides a methanol synthesis reactor fed with the outlet flow of syngas and with the outlet flows of hydrogen which in turn are priorly mixed with at the captured CO2 in a mixer, the resultant flow mixed with the outlet flow of syngas and fed to the reactor, or both fed independently thereof.

Optionally, the aforementioned could be achieved by means of an actuator-controller module communicated with flow sensors in direct contact with the hydrogen flows and the syngas flow, wherein said actuator-controller module computes and transfers the amount of carbon dioxide to be mixed with the hydrogen flows as a function of the syngas flow and its chemical composition thereof.

Waste heat produced in the methanol reactor is used in order to produce steam to feed the SOECs electrolysers of the electrolysers module.

The electrolysers module as above described, allows for it to be directly connected to a renewable energy source (e.g., wind or solar) in a more efficient manner.

More particularly the alkaline and SOECS electrolyser with low dynamic capacity work as the base load of the system, while the PEM electrolysers follow the fluctuating electrical loading because of their rapid response and dynamic capacity. In this way, it is possible to directly connect, for example a wind farm, and work with the dynamic capacity of PEM electrolysers, the alkaline and SOECS working as the base of the system on a constant load.

Preferably, between a 60-90% of the total installed power of the electrolyser's module is for the Alkaline electrolyser, 15-30% of the total power installed is for the PEM electrolyser, 2-10% is for the SOEC electrolyser and 0.5 - 3% for the SOEC co-electrolyser.

Alternatively, a range of 70-85% of the total installed capacity is provided by the Alkaline electrolyser, 15-20% for the PEM electrolyser, 2-6% for the SOEC and 0.3 - 1% is provided by the co-electrolyser.

The above-described proportions for power installed are scalable and it has been discovered to be the most effective when dealing with power fluctuations, availability, and methanol production of the methanol installation.

Regarding to installed power per electrolyser, according to an embodiment of the present invention, preferably the Alkaline electrolyser comprises an installed power of 80-120 MW, a PEM comprising 15-35 MW, a 2-6 MW SOEC, and co-electrolyser of 1-3 MW. Total installed power of the electrolyser module being approximately 98-164MW.

Furthermore, the SOEC and the SOEC co-electrolyser offer a better thermal integrability with the methanol synthesis plant because the waste heat can be converted in steam in the methanol reactor that can be used as input steam for said electrolysers.

Additionally, the possibility of obtaining a flow of nearly pure hydrogen, a flow of syngas and a flow of captured CO2 allows to select the precise proportions of each thereof needed to be introduced in the methanol synthesis reactor so as to allow greater efficiencies and/or methanol yielding in a Fisher-Tropsh process in the methanol synthesis reactor. This greatly improves the efficiency, yielding and versatility of the e-methanol plant, particularly with a renewable energy power source. In other words, availability of the methanol plant is greatly enhanced at the same time methanol reactor efficiency is also improved.

SOECs and PEM electrolysers can work at partial load. Said partial load can be selected as to adapt to the renewable fluctuating power levels and/or to the hydrogen and syngas production needed to be introduced in the reactor in the most efficient manner. Similarly, the CO₂ fed to the electrolysers module and to the mixer can both be selected correspondingly. Thus, the e-methanol installation proposed is significantly flexible and versatile.

Furthermore, the electrolyser module above proposed offers better integrability with renewable energies, and further, allows to directly introduce into a methanol synthesis reactor: a flow of syngas and/or a flow of hydrogen mixed with CO₂ at will, therefore increasing global efficiency and the yielding of the installation.

All the electrolysers need a cooling system to remove the heat produced in the process. Usually, the heat is removed to the ambient using an air or water closed cooling systems. In an embodiment of the invention, it is provided a heat pump which makes use of the heat generated from the electrolysers as an input for said heat pump, in order to obtain water at high temperature mainly for 1) carbon dioxide capture units 2) mud, bark and splinter dryer 3) medium density recycle water 4) MDF (Medium-Density Fiberboards) air preheaters 5) boiler water preheater and 6) feed an absorption system in order to produce cool water to help to the overall facility water cooling system.

The installation may comprise one or more cooling towers fed with a stream of steam from the methanol synthesis reactor.

Optionally, the e-methanol installation could further comprise a heat pump and a heat exchanger interconnected with the electrolyser's module, wherein the heat exchanger is fed with a circulating liquid which absorbs the heat generated from the electrolysers module and said liquid is subsequently passed through the heat pump.

Optionally, heat from the heat pump is transferred to the carbon capture module.

Alternatively, or in addition to the above, heat from the heat pump could be transferred a first absorption unit fed with at least part of the heat generated by the heat pump, wherein the first absorption unit takes here from the first pump and provides cooling to the one or more cooling towers.

In a further embodiment, the installation comprises a burner fed by the exhaust gases of the methanol synthesis reactor, wherein said burner burns said exhaust gases and therefore utilize residual calorific power thereof.

In addition, the installation may further comprise a second absorption unit interconnected with the burner, wherein said second absorption unit takes heat from the burner by a heat exchanging element and provides cooling to the one or more cooling towers.

Note, that part of the heat is used for the purpose of the installation as described but a significant excess heat may also be at the same time used for external purposes. Hence, when referring to heat transferred from modules or units, it may be all or only part of the heat thereof.

In a preferred embodiment, the installation further comprising a scrubber unit interconnected therebetween the boiler and the carbon capture module.

The installation may further comprise comprising a wastewater treatment module receiving residual water from at least the scrubber unit, the water treatment unit, the methanol synthesis unit and the cooling towers.

For the water to be introduced in the electrolyser module, the installation may further comprise a de-mineralization unit for de-mineralizing said water thereof. The de-mineralization unit connected with the wastewater treatment module so that residual water from said de-mineralization unit recirculates back to the water treatment module.

Note that, with the configuration explained above, around 30% of the water is recirculated and hydric footprint is reduced.

The oxygen produced as output of the electrolyser module can be used as either: 1.) feedstock in the water treatment plant, and 2.) for ozone production in an ozone module further comprised in the installation.

Preferably, the carbon capture module is of the enzymatic type, for example, by the carbon-capture kinetics of aqueous potassium carbonate, which reacts with CO₂ and water to form potassium bicarbonate. The bicarbonate solution releases its CO2 at relatively low temperatures (around 75 °C under reduced pressure). For example, a carbonic anhydrase enzyme can be used to catalyze the reaction, speeding up the formation of HCO₃.

Alternatively, the carbon capture module comprises a direct air capture system or DAC. Said DAC captures those diffuse or mobile emissions and that carbon that is present in the atmosphere working under chemical principles similar to those for point-source capture above mentioned. The main difference is that ambient air, although overloaded, is still only 410 ppm.

In a preferred embodiment, the carbon capture module comprises a DAC and an enzymatic capturing module,

### DESCRIPTION OF THE DRAWINGS

To complement the description being made and in order to aid towards a better understanding of the characteristics of the invention, in accordance with a preferred example of practical embodiment thereof, a set of drawings is attached as an integral part of said description wherein, with illustrative and non-limiting character, the following has been represented:

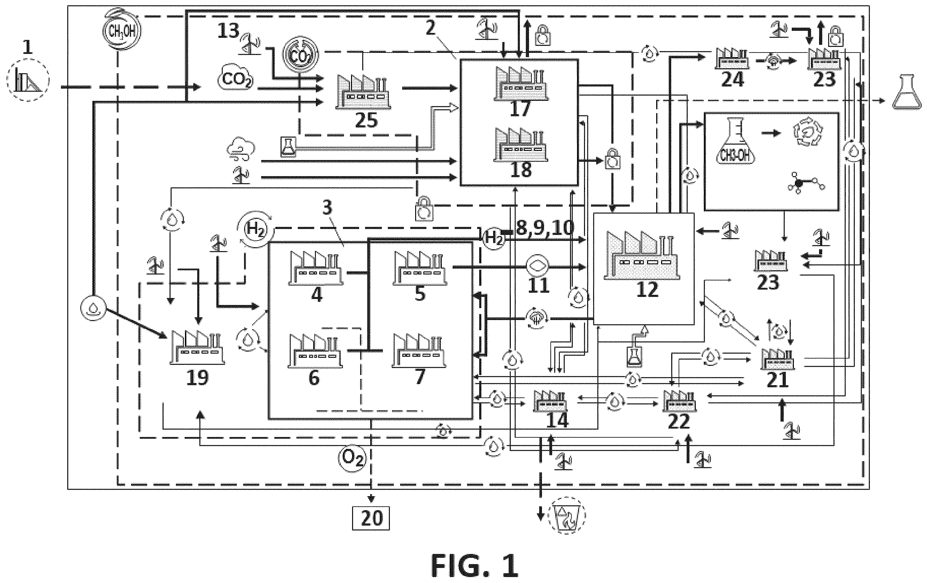

Figure 1.- Shows a schematic representation of the e-methanol production installation according to a preferred embodiment of the invention.

### PREFERRED EMBODIMENT OF THE INVENTION

A detailed description of a preferred exemplary embodiment of the object of the invention is provided below, with the aid of attached Figure 1 described above.

The object of the invention relates to a e-methanol production installation, comprising a boiler (1) fed with biomass, said boiler (1) producing a stream of exhaust gases, a exhaust gases scrubber (25), a carbon capture module (2) operatively connected with the boiler, capturing CO₂ from said stream of exhaust gases, and carbon capture module (2) comprising a direct air capture device (17) taking air from the atmosphere and a enzymatic carbon capture device (18). electrolysers module (3) electrically connected with a renewable power source (13), wherein in a preferred embodiment said power source (13) is a wind farm.

As Figure 1 clearly shows, the electrolyser module (3) in turn comprises:
- a solid oxide co-electrolyser (5) fed with at least part of the captured CO₂, renewable electricity and steam, producing a flow of syngas at 1.5 barg (11),
- a solid oxide electrolyser (7) fed with renewable electricity and steam, producing a first flow of hydrogen at 1.5 barg (8),
- a proton exchange membrane (PEM) electrolyser (6) fed with water and renewable electricity producing a second flow of hydrogen at 30 barg (9),
- an alkaline electrolyser (5) fed with water and renewable electricity producing a third flow of hydrogen at 5 barg (10).

In addition, the methanol production installation further comprises a methanol synthesis reactor (12) fed with the flow of syngas and with the flows of hydrogen (8, 9,10) which in turn are priorly mixed with at least part of the captured CO₂ in the carbon capture module (2). They can be mixed in a specific mixer apparatus or inside the methanol synthesis reactor (12).

In the preferred embodiment described above, the installation further comprises an actuator-controller module (not shown) communicated with flow sensors in direct contact with the hydrogen flows and the syngas flow, wherein said actuator-controller module computes and transfers the amount of carbon dioxide to be mixed with the hydrogen flows (8,9,10) as a function of the syngas flow and its chemical composition thereof.

The methanol production installation obtains three flows of hydrogen, a flow of syngas and a flow of captured CO2 and hence allows to select the precise proportions of each thereof and the CO2 needed to be mixed so as to allow greater efficiencies and/or methanol yielding in a Fisher-Tropsh process in the methanol synthesis reactor (12).

As shown in Figure 1, the installation further comprises a heat pump (14) and a heat exchanger interconnected with the electrolysers module (3), wherein the heat exchanger is fed with a circulating liquid which absorbs the heat generated from the electrolysers module (3) and said liquid is subsequently passed through the heat pump (14).

Furthermore, the methanol production installation comprises one or more cooling towers (21) fed with a stream of steam from the methanol synthesis reactor (12).

To utilize the heat generated in the electrolysers module (3), in a preferred embodiment, the installation further comprises a heat pump (14) and a heat exchanger interconnected with the electrolysers module (3), wherein the heat exchanger is fed with a circulating liquid which absorbs the heat generated from the electrolysers module (3) and said liquid is subsequently passed through the heat pump (14).

Heat from the heat pump (14) is used as input in the carbon capture module (2). In addition, as shown in Figure 1, a first absorption unit (22) is fed with at least part of the heat generated by the heat pump (14), wherein the first absorption unit (22) takes heat from the first pump (14) and provides cooling to the one or more cooling towers (21).

Figure 1 also illustrates that in a preferred embodiment the installation further comprises a burner (24) fed by the exhaust gases of the methanol synthesis reactor (12), wherein said burner (24) burns exhaust gases and utilize residual calorific power thereof.

Subsequently, in the preferred embodiment shown, a second absorption unit (23) interconnected with the burner (24), takes heat from the burner by a heat exchanging elements (not shown) and provides cooling to the one or more cooling towers (21).

Figure 1 further illustrates a scrubber unit (25) interconnected therebetween the boiler (1) and the carbon capture module (2), and a water treatment module (23) receiving residual water from at least the scrubber unit (25) and the cooling towers (21).

For the introduction of water to the electrolysers module (3) a de-mineralization unit (19) is used. Said de-mineralization unit (19) is connected with the wastewater treatment module (23) so that residual water from said de-mineralization unit (19) recirculates to the water treatment module (23).

In a preferred embodiment of the invention the e-methanol production installation comprises a water treatment module or de-mineralization unit (19) fed with raw water and residual water from the wastewater treatment module (23). The wastewater treatment module (23) receive water from the water treatment plant (19), scrubber (25), methanol synthesis unit (12) and cooling tower (9). The water close loop is therefore developed in a way that the maximum amount of water is reused to reduce the hydric footprint.

In the preferred embodiment that has been described, the carbon capture module (2) comprises an enzymatic-type carbon capturing module (17) and a direct air capture module (18).

Finally, the oxygen outlet flow from the electrolysers module (3) is utilized by an ozone production module (20) fed with said oxygen outlet flow.

Alternatively, or additionally, the oxygen outlet flow from the electrolysers module (3) is utilized in the wastewater treatment module (23).

## Claims

1. A biomethanol production installation, comprising:
- a boiler (1) fed with biomass, said boiler (1) producing a stream of exhaust gases,
- a carbon capture module (2) operatively connected with the boiler, capturing CO₂ from said stream of exhaust gases,
- an electrolysers module (3) electrically connected with a renewable power source (13), wherein said electrolyser module (3) comprises at least:
- a solid oxide co-electrolyser (5) fed with at least part of the captured CO₂ and steam, producing a flow of syngas (11),
- a solid oxide electrolyser (7) fed with steam, producing a first flow of hydrogen (8),
- a proton exchange membrane (PEM) electrolyser (6) fed with water and producing a second flow of hydrogen (9),
- an alkaline electrolyser (4) fed with water and producing a third flow of hydrogen (10),
- a methanol synthesis reactor (12) fed with the flow of syngas and with the flows of hydrogen (8, 9,10) which in turn are priorly mixed with at least part of the captured CO₂ in the carbon capture module (2).

2. - The e-methanol production installation of claim 1, further comprising one or more cooling towers (21) fed with a stream of steam from the methanol synthesis reactor (12).

3. - The e-methanol production installation of claim 1, further comprising a heat pump (14) and a heat exchanger interconnected with the electrolysers module (3), wherein the heat exchanger is fed with a circulating liquid which absorbs the heat generated from the electrolysers module (3) and said liquid is subsequently passed through the heat pump (14).

4. - The e-methanol production installation of claim 3, comprising heat conducting elements to transfer heat from the heat pump (14) to the carbon capture module (2).

5. - The e-methanol production installation of claim 2 and 4, further comprising a first absorption unit (22) fed with at least part of the heat generated by the heat pump (14), wherein the first absorption unit (22) takes heat from the first pump (14) and provides cooling to the one or more cooling towers (21).

6. - The e-methanol production installation of claim 1, further comprising a burner (24) fed by the exhaust gases of the methanol synthesis reactor (12), wherein said burner (24) burns said exhaust gases and therefore utilize residual calorific power thereof.

7. - The e-methanol production installation of claim 6, further comprising a second absorption unit (23) interconnected with the burner (24), wherein said second absorption unit (23) takes heat from the burner by a heat exchanging element and provides cooling to the one or more cooling towers (21).

8. - The e-methanol production installation of claim 1, further comprising a scrubber unit (25) interconnected therebetween the boiler (1) and the carbon capture module (2).

9. - The e-methanol production installation of claims 2 and 8 further comprising a wastewater treatment module (23) receiving residual water from at least the scrubber unit (25) and the cooling towers (21).

10. - The e-methanol production installation of claim 9, further comprising a de-mineralization unit (19) connected with the wastewater treatment module (23), wherein residual water from said de-mineralization unit (19) recirculates to the wastewater treatment module (23).

11. - The e-methanol production installation of claim 1, further comprising an ozone production module (20) fed with an oxygen outlet flow from the electrolysers module (3).

12. - The e-methanol production installation of claim 1, further comprising an actuator-controller module communicated with flow sensors in direct contact with the hydrogen flows and the syngas flow, wherein said actuator-controller module computes and transfers the amount of carbon dioxide to be mixed with the hydrogen flows (8,9,10) as a function of the syngas flow and its chemical composition thereof.

## Patentansprüche

1. Anlage zur Produktion von Biomethanol, die Folgendes umfasst:
- einen mit Biomasse beschickten Kessel (1), wobei der Kessel (1) einen Strom von Abgasen erzeugt,
- ein Kohlenstoffabscheidungsmodul (2), das betriebsmäßig mit dem Kessel verbunden ist und CO₂ aus dem Abgasstrom abscheidet,
- ein Elektrolyseur-Modul (3), das elektrisch mit einer erneuerbaren Energiequelle (13) verbunden ist, wobei das Elektrolyseur-Modul (3) mindestens umfasst:
- einen Festoxid-Coelektrolyseur (5), der mit mindestens einem Teil des abgeschiedenen CO₂ und Dampf gespeist wird und einen Strom von Synthesegas (11) erzeugt,
- einen Festoxid-Elektrolyseur (7), der mit Dampf gespeist wird und einen ersten Wasserstoffstrom (8) erzeugt,
- einen Protonenaustauschmembran-(PEM-)Elektrolyseur (6), der mit Wasser gespeist wird und einen zweiten Wasserstoffstrom (9) erzeugt,
- einen alkalischen Elektrolyseur (4), der mit Wasser gespeist wird und einen dritten Wasserstoffstrom (10) erzeugt,
- einen Methanolsynthesereaktor (12), der mit dem Syngasstrom und den Wasserstoffströmen (8, 9, 10) gespeist wird, die ihrerseits zuvor mit mindestens einem Teil des abgeschiedenen CO₂ im Kohlenstoffabscheidungsmodul (2) vermischt werden.

2. Anlage zur Produktion von E-Methanol nach Anspruch 1, die ferner einen oder mehrere Kühltürme (21) umfasst, die mit einem Dampfstrom aus dem Methanolsynthesereaktor (12) gespeist werden.

3. Anlage zur Produktion von E-Methanol nach Anspruch 1, die ferner eine Wärmepumpe (14) und einen Wärmetauscher umfasst, der mit dem Elektrolyseur-Modul (3) verbunden ist, wobei der Wärmetauscher mit einer zirkulierenden Flüssigkeit gespeist wird, welche die vom Elektrolyseur-Modul (3) erzeugte Wärme absorbiert, und die Flüssigkeit anschließend durch die Wärmepumpe (14) geleitet wird.

4. Anlage zur Produktion von E-Methanol nach Anspruch 3, die wärmeleitende Elemente zur Übertragung von Wärme von der Wärmepumpe (14) an das Kohlenstoffabscheidungsmodul (2) umfasst.

5. Anlage zur Produktion von E-Methanol nach Anspruch 2 und 4, die ferner eine erste Absorptionseinheit (22) umfasst, die mit mindestens einem Teil der von der Wärmepumpe (14) erzeugten Wärme gespeist wird, wobei die erste Absorptionseinheit (22) Wärme von der ersten Pumpe (14) aufnimmt und Kühlung für den einen oder die mehreren Kühltürme (21) bereitstellt.

6. Anlage zur Produktion von E-Methanol nach Anspruch 1, die ferner einen Brenner (24) umfasst, der von den Abgasen des Methanolsynthesereaktors (12) gespeist wird, wobei der Brenner (24) die Abgase verbrennt und somit deren Restwärmeleistung nutzt.

7. Anlage zur Produktion von E-Methanol nach Anspruch 6, die ferner eine zweite Absorptionseinheit (23) umfasst, die mit dem Brenner (24) verbunden ist, wobei die zweite Absorptionseinheit (23) über ein Wärmetauscherelement Wärme vom Brenner aufnimmt und Kühlung für den einen oder die mehreren Kühltürme (21) bereitstellt.

8. Anlage zur Produktion von E-Methanol nach Anspruch 1, die ferner eine Wäschereinheit (25) umfasst, die zwischen den Kessel (1) und das Kohlenstoffabscheidungsmodul (2) geschaltet ist.

9. Anlage zur Produktion von E-Methanol nach den Ansprüchen 2 und 8, die ferner ein Abwasserbehandlungsmodul (23) umfasst, das Restwasser von mindestens der Wäschereinheit (25) und den Kühltürmen (21) aufnimmt.

10. Anlage zur Produktion von E-Methanol nach Anspruch 9, die ferner eine Entmineralisierungseinheit (19) umfasst, die mit dem Abwasserbehandlungsmodul (23) verbunden ist, wobei Restwasser aus der Entmineralisierungseinheit (19) zum Abwasserbehandlungsmodul (23) zurückgeführt wird.

11. Anlage zur Produktion von E-Methanol nach Anspruch 1, die ferner ein Ozonproduktionsmodul (20) umfasst, das mit einem Sauerstoffauslassstrom aus dem Elektrolyseurmodul (3) gespeist wird.

12. Anlage zur Produktion von E-Methanol nach Anspruch 1, die ferner ein Stellglied-Steuermodul umfasst, das mit Strömungssensoren in direktem Kontakt mit den Wasserstoffströmen und dem Synthesegasstrom steht, wobei das Stellglied-Steuermodul die mit den Wasserstoffströmen (8, 9, 10) zu mischende Kohlendioxidmenge als Funktion des Synthesegasstroms und seiner chemischen Zusammensetzung berechnet und überträgt.

## Revendications

1. Installation de production de biométhanol, comprenant :
- une chaudière (1) alimentée avec de la biomasse, ladite chaudière (1) produisant un flux de gaz effluents,
- un module de capture de carbone (2) connecté fonctionnellement à la chaudière, capturant du CO₂ à partir dudit flux de gaz effluents,
- un module d'électrolyseurs (3) électriquement connecté à une source d'énergie renouvelable (13), ledit module d'électrolyseurs (3) comprenant au moins :
- un co-électrolyseur à oxyde solide (5) alimenté avec au moins une partie du CO₂ capturé et de la vapeur d'eau, produisant un flux de gaz de synthèse (11),
- un électrolyseur à oxyde solide (7) alimenté avec de la vapeur d'eau, produisant un premier flux d'hydrogène (8),
- un électrolyseur à membrane échangeuse de protons (PEM) (6) alimenté avec de l'eau et produisant un deuxième flux d'hydrogène (9),
- un électrolyseur alcalin (4) alimenté avec de l'eau et produisant un troisième flux d'hydrogène (10),
- un réacteur de synthèse de méthanol (12) alimenté avec le flux de gaz de synthèse et avec les flux d'hydrogène (8, 9, 10) qui sont à leur tour préalablement mélangés avec au moins une partie du CO₂ capturé dans le module de capture de carbone (2).

2. Installation de production d'e-méthanol selon la revendication 1, comprenant en outre une ou plusieurs tours de refroidissement (21) alimentées avec un flux de vapeur d'eau provenant du réacteur de synthèse de méthanol (12).

3. Installation de production d'e-méthanol selon la revendication 1, comprenant en outre une pompe à chaleur (14) et un échangeur de chaleur interconnectés avec le module d'électrolyseurs (3), l'échangeur de chaleur étant alimenté avec un liquide en circulation qui absorbe la chaleur générée à partir du module d'électrolyseurs (3) et ledit liquide étant par la suite amené à passer dans la pompe à chaleur (14).

4. Installation de production d'e-méthanol selon la revendication 3, comprenant des éléments conducteurs de chaleur pour transférer de la chaleur de la pompe à chaleur (14) au module de capture de carbone (2).

5. Installation de production d'e-méthanol selon les revendications 2 et 4, comprenant en outre une première unité d'absorption (22) alimentée avec au moins une partie de la chaleur générée par la pompe à chaleur (14), la première unité d'absorption (22) récupérant de la chaleur de la première pompe (14) et fournissant un refroidissement à la ou les tours de refroidissement (21).

6. Installation de production d'e-méthanol selon la revendication 1, comprenant en outre un brûleur (24) alimenté par les gaz effluents du réacteur de synthèse de méthanol (12), ledit brûleur (24) brûlant lesdits gaz effluents et utilisant par conséquent le pouvoir calorifique résiduel de ceux-ci.

7. Installation de production d'e-méthanol selon la revendication 6, comprenant en outre une seconde unité d'absorption (23) interconnectée avec le brûleur (24), ladite seconde unité d'absorption (23) récupérant de la chaleur du brûleur par un élément d'échange de chaleur et fournissant un refroidissement à la ou les tours de refroidissement (21).

8. Installation de production d'e-méthanol selon la revendication 1, comprenant en outre une unité de lavage (25) interconnectée entre la chaudière (1) et le module de capture de carbone (2).

9. L'installation de production d'e-méthanol selon les revendications 2 et 8 comprenant en outre un module de traitement d'eau résiduaire (23) recevant de l'eau résiduelle provenant au moins de l'unité de lavage (25) et des tours de refroidissement (21).

10. Installation de production d'e-méthanol selon la revendication 9, comprenant en outre une unité de déminéralisation (19) connectée au module de traitement d'eau résiduaire (23), l'eau résiduelle provenant de ladite unité de déminéralisation (19) recirculant vers le module de traitement d'eau résiduaire (23).

11. Installation de production d'e-méthanol selon la revendication 1, comprenant en outre un module de production d'ozone (20) alimenté avec un flux de sortie d'oxygène provenant du module d'électrolyseurs (3).

12. Installation de production d'e-méthanol selon la revendication 1, comprenant en outre un module actionneur-régulateur en communication avec des capteurs de flux en contact direct avec les flux d'hydrogène et le flux de gaz de synthèse, ledit module actionneur-régulateur calculant et transférant la quantité de dioxyde de carbone à mélanger avec les flux d'hydrogène (8, 9, 10) en fonction du flux de gaz de synthèse et de la composition chimique de celui-ci.
